Europäisches Patentamt

(19) European Patent Office    (11) Publication number: **0 012 494**

Office européen des brevets    **B1**

(12) **EUROPEAN PATENT SPECIFICATION**

(45) Date of publication of patent specification: **09.11.83**    (51) Int. Cl.³: **C 12 N  15/00, C 12 P  21/02**

(21) Application number: **79301630.4**

(22) Date of filing: **13.08.79**

(54) **A DNA transfer vector, a microorganism modified by said vector and the synthesis of a eucaryotic protein by the modified microorganism.**

(30) Priority: **11.08.78 US 933035**

(43) Date of publication of application:
**25.06.80 Bulletin 80/13**

(45) Publication of the grant of the patent:
**09.11.83 Bulletin 83/45**

(84) Designated Contracting States:
**BE CH DE FR LU NL SE**

(56) References cited:
**EP - A - 0 001 929**
**EP - A - 0 001 930**
**DE - A - 2 644 432**
**FR - A - 2 281 426**
**FR - A - 2 348 971**
**FR - A - 2 384 024**
**LU - A - 79 174**

**SCIENCE, vol. 198 (4321), 9 December 1977, K. ITAKURA et al. "Expression in Escherichia coli of a Chemically Synthesized Gene for the Hormone Somatostatin", pages 1056—1063**

(73) Proprietor: **THE REGENTS OF THE UNIVERSITY OF CALIFORNIA**
**2200 University Avenue**
**Berkeley, California 94720 (US)**

(72) Inventor: **Rutter, William J.**
**80 Everson Street**
**San Francisco California 94131 (US)**
Inventor: **Goodman, Howard M.**
**3006 Clay Street**
**San Francisco California 94155 (US)**
Inventor: **Baxter, John D.**
**131 San Pablo**
**San Francisco California 94127 (US)**

(74) Representative: **De Minvielle-Devaux, Ian Benedict Peter et al,**
**CARPMAELS & RANSFORD 43, Bloomsbury Square**
**London WC1A 2RA (GB)**

(56) References cited:
**NATURE, vol. 275, 12 October 1978. O. MERCEREAU-FUIJALON et al. "Synthesis of an Ovalbumin-like protein by Escherichia coli K12 Harbouring a recombinant plasmid" pages 505—510**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 012 494**

NATURE, vol. 275, 19 October 1978, A. C. Y. CHANG "Phenotypic expression in E. coli of a DNA sequence coding for mouse dihydrofolate reductase", pages 617—624

NATURE, vol. 276, 21/28 December 1978. P. H. SEEBURG et al. "Synthesis of Growth hormone by bacteria", pages 795—798

PROC. NATL. ACAD. SCIE. USA, vol. 75, no. 8, Aug. 1978, L. VILLA-KOMAROFF et al. "A bacterial clone synthesizing proinsulin", pages 3727—3731

PROC. NATL. ACAD. SCI. USA, vol. 76, no. 1, Jan. 1979, D. V. GOEDDEL et al. "Expression in Escherichia coli of chemically synthesized genes for human insulin", pages 106—110

RESEARCH DISCLOSURE, July 1979, no. 183, ref. no. 18309. "The production of interferon by genetic engineering", pages 361—362

SCIENCE, vol. 196, 17 June 1977 A. ULLRICH et al. "Rat insulin genes: construction of Plasmids containing the coding sequences", pages 1313—1319

NATURE, vol. 270, 8 December 1977, P. H. SEEBURG et al. "Nucleotide sequence and amplification in bacteria of structural gene for rat growth hormone"

**0012494**

A DNA-transfer vector, a microorganism modified by said vector and the synthesis of a eucaryotic protein by the modified microorganism

Reference is made herein to U.S. Serial No. 897,709, filed April 19, 1978 and to U.S. Serial No. 897,710, also filed April 19, 1978. Athough no U.S. patent had issued on these applications prior to the date of the present application, both are available following publication of GB—A—1,565,190 on 16 April, 1980.

Background of the invention

Developments in recombinant DNA technology have made it possible to isolate specific genes or portions thereof from higher organisms, such as man and other mammals, and to transfer the genes or fragments to a microorganism species, such as bacteria or yeast. The transferred gene is replicated and propagated as the transformed microorganism replicates. As a result, the transformed microorganism may become endowed with the capacity to make whatever protein the gene or fragment encodes, whether it be an enzyme, a hormone, an antigen or an antibody, or a portion thereof. The microorganism passes on this capability to its progeny, so that in effect, the transfer has resulted in a new strain, having the described capability. See, for example, Ullrich, A. et al., *Science 196*, 1313 (1977), and Seeburg, P.H., et al., *Nature 270*, 486 (1977). A basic fact underlying the application of this technology for practical purposes is that DNA of all living organisms, from microbes to man, is chemically similar, being composed of the same four nucleotides. The significant differences lie in the sequence of these nucleotides in the polymeric DNA molecule. The nucleotide sequences are used to specify the amino acid sequences of proteins that comprise the organism. Although most of the proteins of different organisms differ from each other, the coding relationship between nucleotide sequence and amino acid sequence is fundamentally the same for all organisms. For example, the same nucleotide sequence which codes for the amino acid sequence of growth hormone in human pituitary cells, will, when transferred to a microorganism, be recognized as coding for the same amino acid sequence.

Abbreviations used herein are given in Table 1.

TABLE 1

| | |
|---|---|
| DNA—deoxyribonucleic acid | A—Adenine |
| RNA—ribonucleic acid | T—Thymine |
| cDNA—complementary DNA | G—Guanine |
| (enzymatically synthesized | C—Cytosine |
| from an mRNA sequence) | U—Uracil |
| mRNA—messenger RNA | Tris—2-amino-2- |
| dATP—deoxyedenosine tri- | hydroxyethyl-1-1, |
| phosphate | 3, propanediol |
| dGTP—deoxyguanosine tri- | EDTA—ethylenediamine |
| phosphate | tetraacetic acid |
| dCTP—deoxycytidine tri- | ATP—adenosine tri- |
| phosphate | phosphate |
| HCS—Human chorionic soma- | TTP—thymidine tri- |
| tomammotropin | phosphate |
| TCA—Trichloroacetic acid | RGH—Rat growth |
| HGH—Human growth hormone | hormone |

The coding relationships between nucleotide sequence in DNA and amino acid sequence in protein are collectively known as the genetic code, shown in Table 2.

TABLE 2
Genetic code

| | | | |
|---|---|---|---|
| Phenylalanine (Phe) | TTK | Histidine (His) | CAK |
| Leucine (Leu) | XTY | Glutamine (Gln) | CAJ |
| Isoleucine (Ile) | ATM | Asparagine (Asn) | AAK |
| Methionine (Met) | ATG | Lysine (Lys) | AAJ |
| Valine (Val) | GTL | Aspartic acid (Asp) | GAK |
| Serine (Ser) | QRS | Glutamic acid (Glu) | GAJ |
| Proline (Pro) | CCL | Cysteine (Cys) | TGK |
| Threonine (Thr) | ACL | Tryptophan (Try) | TGG |
| Alanine (Ala) | GCL | Arginine (Arg) | WGZ |
| Tyrosine (Tyr) | TAK | Glycine (Gly) | GGL |
| Termination signal | TAJ | | |
| Termination signal | TGA | | |

**0 012 494**

Key: Each 3-letter triplet represents a trinucleotide of mRNA, having a 5′ end on the left and a 3′ end on the right. The letters stand for the purine or pyrimidine bases forming the nucleotide sequence.

A=adenine
G=guanine
C=cytosine
T=thymine
X=T or C if Y is A or G
X=C if Y is C or T
Y=A, G, C or T if X is C
Y=A or G if X is T
W=C or A if Z is A or G
W=C if Z is C or T
Z=A, G, C or T if W is C
Z=A or G if W is A
QR=TC if S is A, G, C or T
QR=AG if S is T or C
S=A, G, C or T if QR is TC
S=T or C if QR is AG

J=A or G
K=T or C
L=A, T, C or G
M=A, C or T

An important feature of the code, for present purposes, is the fact that each amino acid is specified by a trinucleotide sequence, also known as a nucleotide triplet. The phosphodiester bonds joining adjacent triplets are chemically indistinguishable from all other internucleotide bonds in DNA. Therefore the nucleotide sequence cannot be read to code for a unique amino acid sequence without additional information to determine the reading frame, which is the term used to denote the grouping of triplets used by the cell in decoding the genetic message.

Many recombinant DNA techniques employ two classes of compounds, transfer vectors and restriction enzymes, to be discussed in turn. A transfer vector is a DNA molecule which contains, *inter alia,* genetic information which insures its own replication when transferred to a host microorganism strain. Examples of transfer vectors commonly used in bacterial genetics are plasmids and the DNA of certain bacteriophages. Although plasmids have been used as the transfer vectors for the work described herein, it will be understood that other types of transfer vector may be employed. Plasmid is the term applied to any autonomously replicating DNA unit which might be found in a microbial cell, other than the genome of the host cell itself. A plasmid is not genetically linked to the chromosome of the host cell. Plasmid DNA's exist as double-stranded ring structures generally on the order of a few million daltons molecular weight, although some are greater than $10^8$ daltons in molecular weight. They usually represent only a small percent of the total DNA of the cell. Transfer vector DNA is usually separable from host cell DNA by virtue of the great difference in size between them. Transfer vectors carry genetic information enabling them to replicate within the host cell, in some cases independently of the rate of host cell division. Some plasmids have the property that their replication rate can be controlled by the investigator by variations in the growth conditions. Plasmid DNA exists as a closed ring. However, by appropriate techniques, the ring may be opened, a fragment of heterologous DNA inserted, and the ring reclosed, forming an enlarged molecule comprising the inserted DNA segment. Bacteriophage DNA may carry a segment of heterologous DNA inserted in place of certain non-essential phage genes. Either way, the transfer vector serves as a carrier or vector for an inserted fragment of heterologous DNA.

Transfer is accomplished by a process known as transformation. During transformation, bacterial cells mixed with plasmid DNA incorporate entire plasmid molecules into the cells. Although the mechanics of the process remain obscure, it is possible to maximize the proportion of bacterial cells capable of taking up plasmid DNA and hence of being transformed, by certain empirically determined treatments. Once a cell has incorporated a plasmid, the latter is replicated within the cell and the plasmid replicas are distributed to the daughter cells when the cell divides. Any genetic information contained in the nucleotide sequence of the plasmid DNA can, in principle, be expressed in the host cell. Typically, a transformed host cell is recognized by its acquisition of traits carried on the plasmid, such as resistance to certain antibiotics. Different plasmids are recognizable by the different capabilities or combination of capabilities which they confer upon the host cell containing them. Any given plasmid may be made in quantity by growing a pure culture of cells containing the plasmid and isolating the plasmid DNA therefrom.

Restriction endonucleases are hydrolytic enzymes capable of catalyzing site-specific cleavage of DNA molecules. The locus of restriction endonuclease action is determined by the existence of a specific nucleotide sequence. Such a sequence is termed the recognition site for the restriction endonuclease. Restriction endonucleases from a variety of sources have been isolated and characterized in terms of the nucleotide sequence of their recognition sites. Some restriction endonucleases hydrolyze the phosphodiester bonds on both strands at the same point, producing blunt ends. Others catalyze hydrolysis of bonds separated by a few nucleotides from each other, producing free single stranded

4

**0012494**

regions at each end of the cleaved molecule. Such single stranded ends are self-complementary, hence cohesive, and may be used to rejoin the hydrolyzed DNA. Since any DNA susceptible of cleavage by such an enzyme must contain the same recognition site, the same cohesive ends will be produced, so that it is possible to join heterologous sequences of DNA which have been treated with restriction endonuclease to other sequences similarly treated. See Roberts, R. J., *Crit. Rev. Biochem. 4*, 123 (1976). Restriction sites are relatively rare, however, the general utility of restriction endonucleases has been greatly amplified by the chemical synthesis of double stranded oligonucleotides bearing the restriction site sequence. Therefore virtually any segment of DNA can be coupled to any other segment simply by attaching the appropriate restriction oligonucleotide to the ends of the molecule, and subjecting the product to the hydrolytic action of the appropriate restriction endonuclease, thereby producing the requisite cohesive ends. See Heyneker, H. L., et al., *Nature 263*, 748 (1976) and Scheller, R. H., et al., *Science 196*, 177 (1977). An important feature of the distribution of restriction endonuclease recognition sites is the fact that they are randomly distributed with respect to reading frame. Consequently, cleavage by restriction endonuclease may occur between adjacent codons or it may occur within a codon.

More general methods for DNA cleavage or for end sequence modification are available. A variety of non-specific endonucleases may be used to cleave DNA randomly, as discussed *infra*. End sequences may be modified by addition of random sequences of dA+dT or dG+dC, to create restriction sites without the need for specific linker sequences.

Using recombinant DNA techniques, as described in application Serial No. 897,710 and by Seeburg, P.H., et al., *supra*, the gene coding for rat growth hormone has been isolated, purified, incorporated into a plasmid transfer vector and transferred to the microorganism *Escherichia coli*, thereby creating a strain of *E. Coli* having the genetic capacity to make rat growth hormone.

The existence of such an organism is of great potential value in the pharmaceutical industry. The growth hormones of mammalian species are closely related in structure and amino acid sequence. Although the hormones from some species are active in other species, this is not always the case. Most non-primate growth hormones, for example, are ineffective in humans. The hormone is synthesized by the pituitary gland in tiny quantities. Until now, supplies of any growth hormone, and especially human growth hormone, have been extremely small. Clinical and investigational availability is very limited. The means for producing growth hormones of humans and animal species is of enormous significance in pharmacology, human medicine, veterinary medicine and animal husbandry. Human growth hormone has medical utility in the treatment of several defects of human growth and development. The hormone may be obtained from human cadavers and is used in the treatment of severe cases of pituitary dwarfism. The cost of treatment is currently estimated to be $5000.00 per year per patient. More than 1000 new patients require treatment each year although the great expense limits availability of treatment to the most severe cases. If an adequate supply of hormone were available at reasonable cost, an estimated additional 10,000 patients with less severe growth hormone deficiency of with certain other growth problems would be treatable. In addition, there is evidence that human growth hormone may be useful for treating a number of other medical problems such as gastrointestinal hemmorhage, fracture healing, metabolic bone disease and wound healing. There is presently insufficient hormone to rigorously determine the utility of such therapy.

Animal growth hormones have commercial utility, particularly in the case of animals raised for slaughter, where rapid maturation and large size are desirable attributes, and where maximum conversion of feed into protein is economically beneficial. By transferring the growth hormone gene to a microorganism, it is possible to develop a fermentation technology capable of producing growth hormone in the desired quantities, from the desired source, at a fraction of the current cost. However, it was observed that when the growth hormone gene was initially transferred to the microorganism, it was not expressed, detectable levels of growth hormone synthesis by the microorganism were not observed.

Using recombinant DNA techniques, as described in US application Serial No. 897,709 and by Ullrich, A., *supra*, the gene coding for rat insulin has been isolated, purified, incorporated into a plasmid transfer vector and transferred to the microorganism *Escherichia coli*, thereby creating a strain of *E. coli* having the genetic capacity to make rat insulin. The existence of such an organism is of great potential value in the pharmaceutical industry and in the field of medicine. Heretofore, diabetics requiring insulin have been dependent upon insulin extracted from the pancreas glands of animals obtained at slaughter. However, the world wide demand for insulin is projected to exceed the supply. Furthermore, some patients develop allergic reactions to bovine or porcine insulin commonly used. Therefore a means for manufacturing human insulin in quantity is highly desirable. By transferring the insulin gene to a microorganism, it is possible to develop a fermentation technology capable of producing insulin in the desired quantities, from the desired source. However, it was observed that the insulin gene, as initially transferred to the microorganism, was not expressed.

The term "expression" is used in recognition of the fact that an organism seldom if ever makes use of all its genetically endowed capabilities at any given time. Even in relatively simple organisms such as bacteria, many proteins which the cell is capable of synthesizing are not synthesized, although they may be synthesized under appropriate environmental conditions. When the protein product, coded

5

by a given gene, is synthesized by the organism, the gene is said to be expressed. If the protein product is not made, the gene is not expressed. Normally, the expression of genes in *E. coli* is regulated as described generally, *infra*, in such manner that proteins whose function is not useful in a given environment are not synthesized and metabolic energy is conserved. A number of hypotheses could be advanced to account for the initial failure to observe expression eucaryotic genes in *E. coli*. Many differences between procaryotic and eucaryotic gene expression and control have been noted. The discovery of hitherto unsuspected non-coding intervening sequences which interrupt the coding sequences of many eucaryotic genes, has raised the possibility of yet other structural differences which might adversely affect the ability of procaryotes to express eucaryotic DNA. See, e.g. Tilghman. S.M., et al., *Proc. Nat. Acad. Sci. USA 74*, 4406 (1977), and Jeffreys, A. J. & Flavell, R. A. *Cell 12*, 1097 (1977). It is possible that the eucaryotic DNA has nucleotide sequences which would be misinterpreted by the bacterial transcription or translation enzymes. While the universality of the genetic code is an accepted fact, the observation that certain codons seem to be preferred in eucaryotic DNA is not satisfactorily explained. It is well known that the controlling elements which regulate the timing and amount of expression of genes in living cells are much different in the cells of higher eucaryotes, such as the rat or man, then they are in procaryotes such as *E. coli*.

The means by which gene expression is controlled in *E. coli* is well understood, as the result of extensive studies over the past twenty years. See, generally, Hayes, W., *The Genetics of Bacteria and their Viruses*, 2d edition, John Wiley & Sons, Inc., New York (1968), and Watson, J. D. *The Molecular Biology of the Gene*, 3d edition, Benjamin, Menlo Park, California (1976).

These studies have revealed that several genes, usually those coding for proteins carrying out related functions in the cell, are found clustered together in continuous sequence. The cluster is called an operon. All genes in the operon are transcribed in the same direction, beginning with the codons coding for the N-terminal amino acid of the first protein in the sequence and continuing through to the C-terminal end of the last protein in the operon. At the beginning of the operon, proximal to the N-terminal amino acid codon, there exists a region of the DNA, termed the control region, which includes a variety of controlling elements including the operator, promoter and sequences for the ribosomal binding sites. The function of these sites is to permit the expression of those genes under their control to be responsive to the needs of the organism. For example, those genes coding for enzymes required exclusively for utilization of lactose are not expressed unless lactose or an analog thereof is actually present in the medium. The control region functions that must be present for expression to occur are the initiation of transcription and the initiation of translation. Expression of the first gene in the sequence is initiated by the initiation of transcription and translation at the position coding for the N-terminal amino acid of the first protein of the operon. The expression of each gene downstream from that point is also initiated in turn, at least until a termination signal or another operon is encountered with its own control region, keyed to respond to a different set of environmental cues. While there are many variations in detail on this general scheme, the important fact is that, to be expressed in a procaryote such as *E. coli*, a gene must be properly located with respect to a control region having initiator of transcription and initiator of translation functions.

It has been demonstrated that genes not normally part of a given operon can be inserted within the operon and controlled by it. The classic demonstration was made by Jacob, F., et al., *J. Mol. Biol. 13*, 704 (1965). In that experiment, genes coding for enzymes involved in a purine biosynthesis pathway were transferred to a region controlled by the lactose operon. The expression of the purine biosynthetic enzyme was then observed to be repressed in the absence of lactose or a lactose analog, and was rendered unresponsive to the environmental cues normally regulating its expression.

In addition to the operator region regulating the initiation of transcription of genes downstream from it, there are known to exist codons which function as stop signals, indicating the C-terminal end of a given protein. See Table 2. Such codons are known as termination signals and also as nonsense codons, since they do not normally code for any amino acid. Deletion of a termination signal between structural genes of an operon creates a fused gene which could result in the synthesis of a chimeric protein consisting of two amino acid sequences coded by adjacent genes, joined by a peptide bond. That such chimeric proteins are synthesized when genes are fused was demonstrated by Benzer, S., and Champe, S. P., *Proc. Nat. Acad. Sci. USA 48*, 114 (1962).

A recombinant plasmid transfer vector has been constructed containing that portion of the lactose operon comprising the operator, promoter and substantial portion of the structural gene for the first enzyme in the operon, beta-galactosidase. See Polisky, B., et al., *Proc. Natl. Acad. Sci. USA 73*, 3900 (1976). The parent plasmid was designated pSF2124, a derivative of ColE1, So, M., et al., *Mol. Gen. Genet. 142*, 239 (1975). The lactose operon region was derived from lambda *plac* 5, Shapiro, J., et al., *Nature 224*, 768 (1969). Joining the 28S ribosomal DNA of *Xanopus laevis* immediately subsequent to the beta-galactosidase gene fragment resulted in the production of RNA capable of hybridizing with *Xenopus* 28S DNA in the presence of an inducer of the lactose operon. In addition, it was reported that certain cultures produced a form of beta-galactosidase of higher molecular weight than the wild type enzyme, suggesting the synthesis of a chimeric protein.

Expression of lower eucaryotic genes from yeast transferred to *E. coli* has been inferred from the experiments of Ratzkin, B. and Carbon, J., *Proc. Nat. Acad. Sci. USA 74*, 487 (1977) and of Struhl, D. and Davis, R. W., *Proc. Nat. Acad. Sci. USA 74*, 5255 (1977).

In those experiments, certain cloned yeast genes were able to complement a deficiency in the transferred bacteria, although other yeast genes were unable to do so. The transferred yeast genes included their own control region. The means of achieving expression of those transferred yeast genes which were expressed therefore appears to depend upon the rare occurance of transfer of the entire structural gene together with its control region. Furthermore, there may exist only a limited set of yeast control regions capable of functioning within a bacterial cell.

Summary of the invention

The present invention includes a DNA transfer vector containing an expressible eucaryotic gene, a microorganism containing and expressing a eucaryotic gene, and methods for making said plasmid and microorganism. The term expression transfer vector is used to denote a transfer vector comprising a eucaryotic gene so located with respect to a control region, that expression of the gene can occur in a microorganism containing the transfer vector. The essential feature of an expression transfer vector is that the heterologous gene, whose expression is desired, is joined to a procaryotic control fragment. The control fragment, as the term is used here, may consist of no more than that part of the control region providing for initiation of transcription and initiation of translation, and may additionally include a portion of a structural gene. The heterologous DNA must be joined to the control fragment such that their reading frames are in register. The reading frames are defined as in register if the same group of nucleotides by threes results in correct amino acid assignments in both the control fragment and the heterologous gene. If the heterologous gene is joined in such a way that the normal reading frame in the gene is maintained, expression of genes controlled by the control region will result in the synthesis of a chimeric protein in which the normal gene product is fused with the heterologous gene product.

Within this framework, there are two basic strategies embodied in the present invention. By connecting the eucaryotic gene to a gene for a protein normally compartmentalized or protected from intracellular degradation in some manner, the stability of the chimeric protein may be enhanced. Preferably, the chimeric protein is transported through the cell membrane and excreted into the growth medium. In that case, there is an added advantage that the extracellular protein will be easier to purify. The second general strategy is to link the eucaryotic gene to a gene which has desirable genetic properties, such as a gene whose control region has advantageous properties which may facilitate construction of the expression transfer vector, its maintenance in the host cell or the rate of synthesis of the chimeric protein.

The first general strategy is exemplified in the case of rat growth hormone. The rat growth hormone gene was connected to the *E. coli* beta-lactamase gene involved in conferring ampicillin resistance. When the described transfer vector is used to transform a microorganism, the latter can synthesize a chimeric protein having the amino acid sequence of a portion of beta-lactamase connected to the amino acid sequence for rat pre-growth hormone, pre RGH. Similarly, the rat pre-proinsulin gene was fused to the *E. coli* beta-galactosidase gene on a plasmid. A chimeric protein was expressed.

The basic requirements for functionality of the present invention are as follows:— There must be generated a cleavage site within the gene normally expressed under a control region. Similarly, there must be generated a cleavage site in the heterologous DNA at or near but outside the nucleotide sequence coding for the N-terminal amino acid of the protein whose synthesis is desired. Joining of the procaryotic gene DNA with the heterologous DNA must occur such that the reading frame of the procaryotic gene is the same as the reading frame of the heterologous DNA. Most conveniently, cleavage will be accomplished with restriction endonucleases, the restriction sites chosen will be sensitive to the same restriction enzyme and will be in register with respect to their respective reading frames. Therefore, when joined, both the procaryotic DNA and the heterologous DNA have the same reading frame.

It is entirely feasible to join randomly-cleaved procaryotic and heterologous DNAs, then selecting for the desired result by appropriate criteria. The chance of in register joining is 1/3, the chance of correct orientation is 1/2, so that potentially expressible fused genes should occur in 1/6 cases, a result well within the power of presently known selection methods. Therefore, with the methods of the present invention, it would be possible to join any eucaryotic gene or gene fragment to a suitably chosen procaryotic gene or control fragment thereof, to construct an expression transfer vector.

Detailed description of the invention

Several conditions must be satisfied in order to transfer a segment of heterologous DNA to a locus on a transfer vector influenced by a control region that expression of the heterologous DNA occurs yielding a chimeric protein. First, it is necessary to choose a functional gene on the transfer vector into which the heterologous DNA will be inserted. The choice of operon fragment will be governed by considerations of convenience in the light of overall goals. For example, the use of the ampicillin resistance operon, coding for the enzyme beta-lactamase, is attractive because the operon is already present on many plasmids commonly used in recombinant DNA work and also because of the fact that substantial amounts of the enzyme are found in the periplasmic space and released by spheroplast formation. If the same behavior is observed with the chimeric protein, transfer to the periplasmic space or even secretion from the cell sequesters the protein from the internal milieu of the cell, and may help stabilize the protein. In addition, its purification would be greatly simplified. The *lac* operon is attractive because it has been extensively studied genetically and a wide variety of mutants is available with which to modify the rate of gene expression under control of the *lac* operator. Other considerations include the possibility of the biosynthesis of chimeric proteins capable of being readily cleaved by enzymic or chemical means, to separate the desired segment from the chimeric protein.

The gene to which the heterologous DNA is to be joined may be cleaved by non-specific or specific means. Non-specific cleavage is accomplished by any of a variety of known endonucleases, or by sonication. Since cleavage is essentially random, unique molecules will result. Whether an expression plasmid ultimately results must then be determined by appropriate microbiological screening and selection techniques. Any particular desired outcome will be rare among many possible outcomes. Therefore the use of non-specific cleavage places more of a burden on the selectivity and sensitivity of the selection and screening techniques.

Preferably, specific cleavage is employed, using a selected restriction endonuclease acting at a known restriction site. Preferably there should be another restriction site, either for the same or a different restriction endonuclease, which will permit a segment of the plasmid to be removed and replaced by a new segment containing the heterologous DNA.

Regardless of how the procaryotic gene is initially cleaved, it is possible to modify the ends by adding or subtracting nucleotides for the purpose of generating or altering restriction site specificity at the ends, or to facilitate blunt-end ligation. A variety of exonuclease enzymes are known, which hydrolyze phosphodiester bonds at 3' and 5' termini. Many restriction endonucleases make staggered cuts in double-stranded DNA, resulting in short single-stranded tails at the cleaved ends. The unpaired, single-stranded regions can be removed, if desired by exonucleases, such as S1 nuclease, having the proper substrate specificity. Alternatively, fully paired ends may be generated by extending the complementary strand by the synthesis of a short segment of new DNA to fill the unpaired region. DNA polymerase may be used for this purpose. Still another alternative is to add nucleotides randomly. For example, random addition of deoxyguanylic acid (dG) and deoxycytidylic acid (dC) can generate a sequence GGCC, reading from 5' to 3' left to right. This is the sequence of the *Hae*III recognition site. The use of random nucleotide additions can therefore generate new restriction sites, although they will be randomly located with respect to the original end and with respect to reading frame.

A powerful and specific technique for modifying ends is provided by the synthesis of linker oligonucleotides as described by Scheller, R. H. et al., *Science 196*, 177 (1977). Any desired nucleotide sequence can be added to the ends of DNA molecules by this technique. Therefore any desired restriction site could be added. Longer nucleotide sequences could be added, in principle. It seems likely that it will be possible to synthesize the control region sequences that control initiation of transcription and translation and that such a sequence could be added directly to the heterologous DNA.

The same techniques used to modify the ends of the procaryotic operon fragment are also applicable to the heterologous DNA. It will be understood that such treatments should not alter or modify the coding sequence for the protein to be synthesized. The heterologous DNA fragment to be transferred should not contain the sequence of a procaryotic control function and preferably contains appropriate restriction sites lying outside the region to be expressed. Preferably these sites will be susceptible to the same restriction endonucleases used to excise a region of the plasmid into which the heterologous DNA is to be transferred. The foregoing requirement is not absolute and can be circumvented if necessary by resort to such techniques as the use of chemically synthesized linkers, and blunt end ligation. It is essential that the cleavage points of the operon and of the heterologous DNA to be attached thereto, be in register with respect to the reading frame. This condition is necessary in order to provide for correct reading of the heterologous codons. For example, if the break point in the operon is between triplets then the corresponding break in the heterologous DNA must also be between triplets. If the break point in the operon is after the first nucleotide from the 3' end of a triplet, the break point of the heterologous DNA must be two nucleotides from the 5' end of a triplet, and so on.

It is additionally necessary that the heterologous DNA must be inserted in the proper orientation. The direction of transcription of the heterologous gene must be the same as that of the operon. Otherwise, the heterologous DNA information will be read in reverse and will be meaningless. In some cases it may be possible to determine whether the insertion is properly oriented. In others, it may be necessary to select, after the fact for the proper orientation, which should occur with 50% probability.

It is also necessary to provide for a selection system to identify, among the products of a DNA-ligase catalyzed reaction, those recombinant transfer vectors containing the correct combination of polynucleotide fragments.

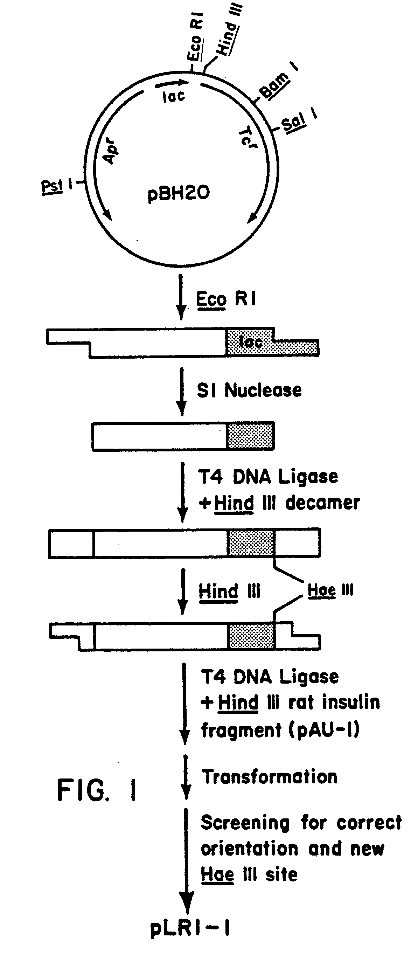

The generally described procedures will be better understood by a general description of specific techniques employed in the present invention. Figure 1 diagrams the steps used to construct a plasmid containing most of the rat proinsulin gene joined to the control region and a short portion of the structural gene of beta-galactosidase. In Figure 1—4, double-stranded DNA is represented by a single line, and plasmid DNA, in the closed loop form, is represented by a circle. The approximate locations of relevant restriction sites are indicated. The relevant genes, lactose operon (*lac*), ampicillin resistance

(Ap$^r$) coding for beta-lactamase, and tetracycline resistance (Tc$^r$) are shown as arcs drawn parallel to their approximate locations relative to the restriction sites and to each other. Arrowheads indicate the direction of transcription for each gene.

A plasmid, pBH20, having incorporated a lactose operon fragment, λplac5 cleaved at a HaeIII site near the control region, was previously constructed. See Itakua, K., et al., Science 198, 1077 (1977). Treatment of pBH20 with EcoRI restriction endonuclease produced a linear plasmid molecule with single-stranded, self-complementary (cohesive) ends. The single-stranded ends were digested away by S1 nuclease treatment. The resulting blunt-ended molecule was joined by DNA ligase to a synthetic nucleotide decamer containing a HindIII restriction site. (Blunt-end ligation has been reported by Sgaramella, V., et al., Proc. Nat. Acad. Sci. USA 67, 1468 (1970). The purpose of adding the HindIII site was to enable a specific joining reaction with a HindIII site on the proinsulin gene fragment. Joining by DNA ligase results in higher yields of joined molecules if the ends are cohesive.

The proinsulin gene fragment, pAU-1, contained all of the proinsulin structural gene except the first 2 N-terminal amino acids, and was flanked by HindIII linkers. From knowledge of the nucleotide sequence of the lac gene fragment of pAU-1 and of the linkers permitted, it could be shown that the proinsulin gene would be in register with respect to reading frame, with the lac fragment. The lac sequence in the region of the EcoRI cleavage was:

5'-GGATTCACTGG-3'

3'-CCTAAGTGACCTTAA-5'

S1 nuclease digestion should remove only the terminal unpaired TTAA sequence. Sometimes, S1 nuclease digests additional residues from the chain, an undesirable result since the expected reading frame registration may be lost. In the present instance, if the S1 digestion was proper, the addition of the HindIII linker would generate an HaeIII site at the junction of the linker and the lacDNA:

lac              HindIII linker

5'-GGATTCACTGGCCAAGCTTGG

3'-CCTAAGTGACCGGTTCGAACC

HaeIII site

Therefore it was possible to test for the correct product independently of any expression, by testing for the new HaeIII site.

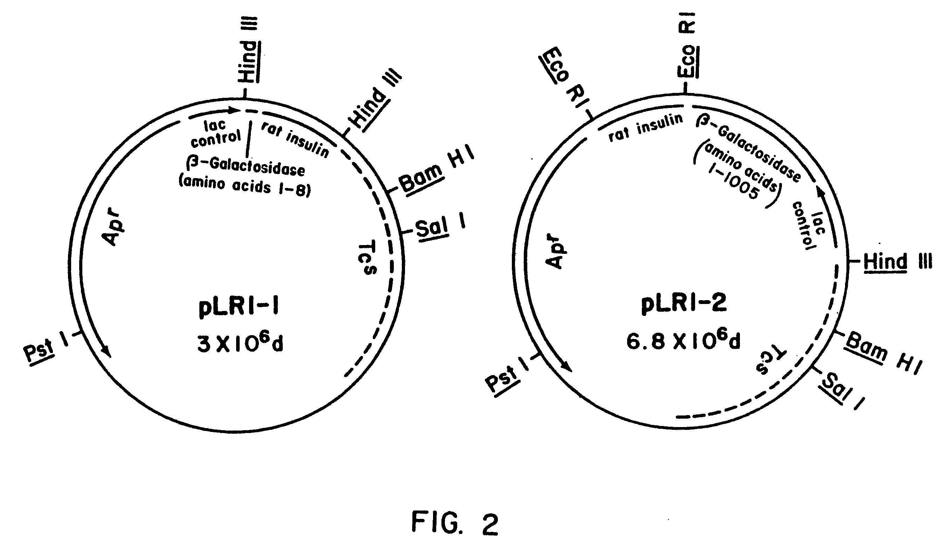

The proinsulin fragment and the lac gene fragment were each treated with HindIII to generate cohesive ends and joined by the action of T4 DNA ligase. E. coli cells were transformed with the ligase products. Transformants were selected by their ability to grow in the presence of ampicillin. Transformants having an inserted proinsulin gene fragment were not resistant to 20 μg/ml tetracycline. The pAU-1 sequence in pLRI-1 is flanked by HindIII sites, which were used for its insertion. Location of the pAU-1 sequence on pLRI-1 is such that the heterologous DNA interrupts a portion of the promoter for the tetracycline resistance gene, thereby lowering its rate of expression. However, if the pAU-1 sequence is oriented such that the poly dA:dT portion of the gene is adjacent the remaining promoter sequence there will be a low level of expression of the tetracycline resistance gene. Consequently, pAU-1 inserted in desired orientation can confer resistance to low levels of tetracycline, 5 μg/ml, on bacteria carrying this transfer vector. This fact was used to select correctly oriented insertion. Finally, the transformed colonies were screened for the existence of a new HaeIII site by isolation of plasmids, treatment with HaeIII and analysis of the size distribution of the HaeIII-produced fragments on gel electrophoresis. A plasmid satisfying all criteria was chosen for further studies of expression and designated pLRI-1, shown schematically in Figure 2.

Two assays for expression of the proinsulin gene of pLRI-1 were employed. In the first, the sizes of proteins synthesized by pLRI-1 transformed cells were compared on sodium dodecyl sulfate (SDS)-acrylamide gel electrophoresis with those synthesized by pBH20 transformed cells. The size region of the expected chimeric protein was crowded with many bands, so that any chimeric protein could not be detected. A second attempt was made using transformed minicells. The latter are produced in the course of growth by the mutant E. coli P678—54. See Adler, H. I., et al., Proc. Nat. Acad. Sci. USA 57, 321 (1966). In addition to normal cell division, P678—54 cells undergo aberrant division which results in formation of minicells, much smaller than the parent cells and lacking parental cell DNA. Consequently, minicells are not endowed with capability to carry out DNA-directed protein synthesis. Otherwise the minicells appear normal, have a normal appearing cell wall and cytoplasmic contents apparently identical with normal cell cytoplasm. When transformed by a plasmid, the strain produces minicells containing copies of the plasmid. Such minicells have a limited capacity to express plasmid genes. The number of possible proteins made by such minicells is greatly restricted compared to normal cells and this fact forms the basis for analysis of plasmid-coded proteins on SDS-acrylamide gel electrophoresis. See Meagher, R. B., et al., *Cell 10*, 521 (1977). Results of such analysis with pLRI-1-transformed minicells were also negative.

The results so far obtained with pLRI-1 indicate a low level of detectable expression with this plasmid. The chimeric protein is a novel substance with a new amino acid sequence. At the present time, it is impossible to predict details of the folding configuration or functional properties of proteins, from their amino acid sequence. The response of the cell to the new protein cannot be predicted. The chimeric protein may be unstable in the intracellular milieu for unknown reasons. The protein may be degraded by intracellular proteases, or it may associate with insoluble cell components. The length of beta-galactosidase gene contributing to the chimeric protein does not appear to be the sole factor, since expression of ovalbumin attached to the same length of beta-galactosidase has been observed. What appears to be critical is the nature and properties of the specific chimeric protein expressed. Consequently since pLRI-1 is known to be properly constructed with respect to reading frame and orientation, it is expected that an appropriate test will ultimately reveal the existence of expression of the proinsulin gene.

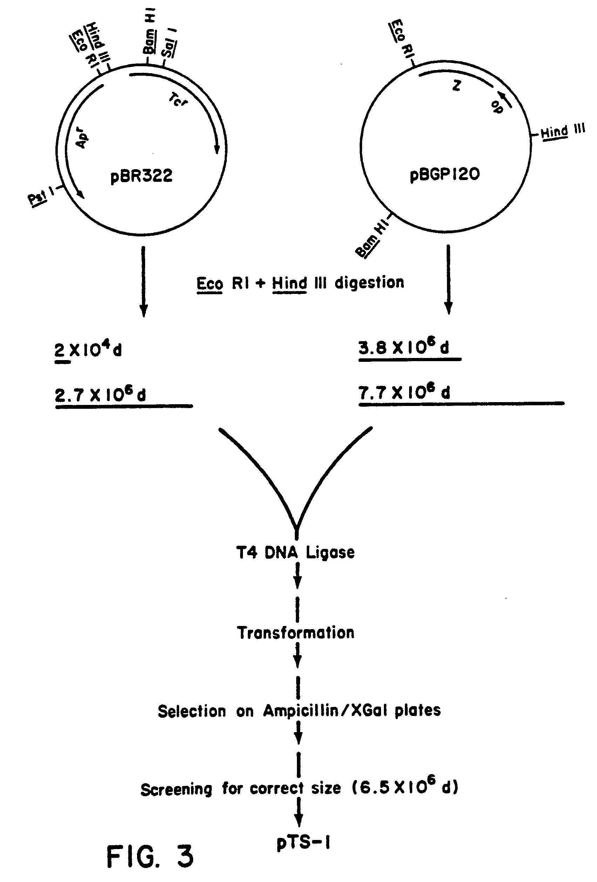

In a second type of study, a larger heterologous DNA was used, comprising the entire proinsulin gene as well as 13 amino acids of the pre insulin sequence. Figure 3 shows the construction scheme of the plasmid into which the rat pre-proinsulin gene was inserted. Plasmid pBGP120 (Polinsky, B., et al., *supra*) was used as the source of a *lac* operon fragment coding for approximately 1000 amino acids. Plasmid pBR322, Bolivar, F., et al., *Gene 2* 95 (1977) was used as a carrier for the *lac* operon fragment by virtue of its smaller size and well-characterized ease of handling during transformation, growth and isolation. Both plasmids were cleaved with combined *Hind*III and *Eco*RI restriction endonucleases, yielding two fragments from each plasmid, with molecular weights as shown in Figure 3. The mixed fragments were joined with T4 DNA ligase, yielding both parental plasmids, single cross-products and possibly more complex forms. All possible combinations could be distinguished by size by agarose gel electrophoresis.

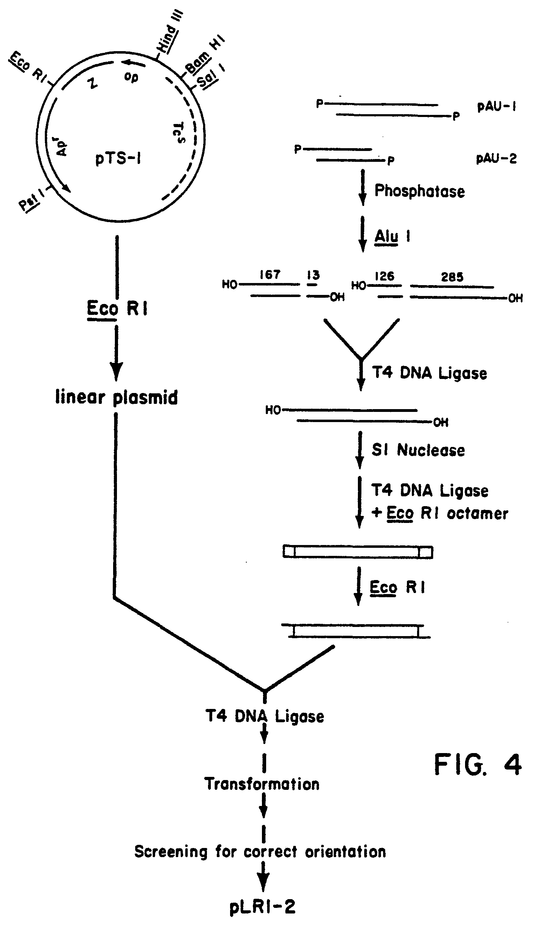

Transformants were selected on XGal plates (Miller, J., *Experiments in Molecular Genetics*, Cold Spring Harbor Laboratory, 1972) containing ampicillin. Colonies having beta-galactosidase are blue on XGal plates and only those strains that a Ap$^r$ will grow at all. In order to test for correct plasmid size, several blue colonies were picked, small cultures were grown from each, the plasmids reisolated and screened for proper size, $6.5 \times 10^6$ daltons, on agarose gel electrophoresis. To further confirm the structure of the desired plasmid, the sizes of the combined *Hind*III, *Eco*RI digestion fragments were measured by polyacrylamide gel electrophoresis. The resulting plasmid, designated pTS-1, is diagrammed in Figure 4.

In order to construct an insulin gene fragment coding for the entire proinsulin sequence, two previously cloned fragments whose nucleotide sequences were known, pAU-1 and pAU-2 were combined. See Ullrich, A., *supra*. The two fragments overlap in their sequences and have a common *Alu*I site in the overlapping region which was used as the point of cleavage. Pretreatment with alkaline phosphatase removed their terminal phosphate groups. *Alu*I cleavage produced blunt ends and since the cleavage site was in the region of common sequence, blunt-end ligation would join the fragments and reconstitute the entire gene. The larger two fragments whose joining was desired, were purified by gel electrophoresis prior to the ligase treatment. After ligase treatment, the three possible joined products were fractionated by gel electrophoresis, and the desired middle-sized fragment was isolated. Both pAU-1 and pAU-2 were originally inserted via *Hind*III linkers. The remaining ends of the combined DNA molecule have *Hind*III-type cohesive ends. The combined molecule was therefore readily inserted at the *Hind*III site of pBR322, and transformants carrying the combined plasmid could be used to provide ample supplies of the combined insulin gene for the remaining steps of the procedure.

The combined insulin gene was released from the plasmid by *Hind*III digestion and purified. The end groups were modified by treatment with S1 nuclease to remove the single-stranded ends and further modified by addition of a synthetic linker having the sequence:

TGAATTCA
ACTTAAGT

providing *Eco*RI specificity. See Greene, P. J., et al., *J. Mol. Biol. 99*, 237 (1975). The modified DNA was then treated with *Eco*RI and inserted into the *Eco*RI site of pTS-1. Correct reading frame is preserved for this *Eco*RI site insertion in the beta-galactosidase gene, see Polisky, B., et al., *supra*. Screening for correct orientation of the inserted insulin gene was carried out by taking advantage of a *Hae*III site in the inserted insulin gene that is asymmetrically placed with respect to other *Hae*III sites on the plasmid. Correct orientation produces a predictable size distribution upon *Hae*III digestion and gel electrophoresis. The resulting plasmid having a correctly inserted insulin gene was designated pLRI-2 and is diagrammed in Figure 2. A similar strategy may be devised for constructing a human insulin expression plasmid, operating within the general principles described.

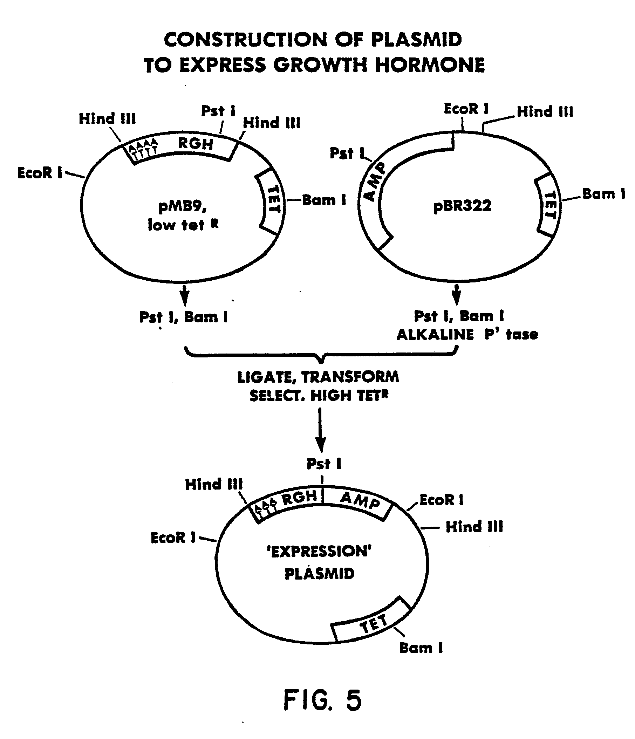

The reaction sequence for joining the rat growth hormone gene to the beta-lactamase gene provides an additional example of the foregoing generally described process. Reference is made to Figure 5 which provides a diagrammatic outline of stages of the process. In Figure 5 double stranded DNA is represented by a single line and plasmid DNA, in the form of a closed loop, is represented by a circle. The approximate locations of the relevant restriction sites are indicated. The relevant genes, rat growth hormone (RGH), ampicillin resistance (AMP) coding for beta-lactamase, and tetracycline resistance (TET), are shown as boxes occupying their approximate locations with respect to the restriction sites and to each other.

The original recombinant plasmid carrying the RGH gene was derived as described in U.S. Serial No. 897,710 and by Seeburg, P. H., et al., *Nature 270*, 486 (1977), and is designated pRGH-1. The rat growth hormone nucleotide sequence contained in pRGH-1 has been determined and has been correlated with those portions of the rat growth hormone amino acid sequence that are known. The plasmid RGH nucleotide sequence is approximately 800 base-pairs long and includes nucleotide coding for the 26 amino acid prepeptide as well as 29 nucleotides comprising part of the 5' untranslated region of growth hormone mRNA. In addition, the sequence contains approximately 100 nucleotides comprising all of the 3' untranslated region. A recognition site for the restriction enzyme *Pst*I exists between the 23rd and 24th amino acids of the pre-RGH sequence. A *Pst*I site also exists in the ampicillin resistance gene of plasmid pBR322 and of pMB9. The RGH sequence in pRGH-1 is flanked by *Hind*III sites, which were used for its insertion.

The 800 base-pair RGH gene was isolated by *Hind*III digestion of pRGH-1, and was purified by polyacrylamide gel electrophoresis. Plasmid pMB9, which contains a single *Hind*III site in the promoter of the tetracycline resistance gene, was cleaved with *Hind*III and treated with bacterial alkaline phosphatase to prevent self ligation in subsequent steps. See, Ullrich, A., et al, *supra*. A mixture of the cleaved plasmid DNA and the RGH-DNA segment was treated with DNA ligase to produce a recombinant plasmid designated pMB9 (RGH), as diagrammed in Figure 5. The resulting plasmids were used to transform *E. coli* and recovered by selection of colonies resistant to 5 $\mu$g/ml tetracycline.

Two possible orientations of the RGH sequence were possible. The desired orientation could be distinguished by preparation of radioactively labeled plasmids from single colony isolates followed by digestion with a combination of *Eco*RI and *Pst*I endonucleases. The size of labeled fragments produced could be determined by gel electrophoresis. Orientation of the RGH segment in the opposite direction would have the result of placing the *Pst*I site closer to the *Eco*RI site yielding a smaller fragment upon digestion with the combined restriction enzymes than that expected for the desired orientation.

The following purification of the correctly oriented pMB9 (RGH) the plasmid was digested with a combination of *Pst*I and *Bam*HI endonucleases. Plasmid pBR322, shown in Figure 5, was similarly digested with *Pst*I and *Bam*HI. In pBR322, the *Pst*I site occurs between amino acids 182 and 183 from the N-terminus of the beta-lactamase gene, counting from the first of 23 amino acids of the beta-lactamase precursor protein. The *Bam*HI site is in the tetracycline resistance gene in exactly the same place as in pMB9, since this region of pBR322 was derived from pMB9, see Bolivar, R., *supra*.

After digestion pBR322 was treated with bacterial alkaline phosphatase to prevent self ligation. The fragments were then joined, via DNA ligase, to the cleaved pMB9 (RGH). The resulting plasmid structure is shown in Figure 1, designated pExRGH, to indicate its role as an expression plasmid. After transformation with the ligase reaction products, transformed bacteria were selected for resistance to high levels of tetracycline, 20 $\mu$g/ml. Since the parent plasmid, pMB9 (RGH) was resistant only to 5 $\mu$g/ml tetracycline and pBR322 was prevented from self ligation by phosphatase treatment, as well as electrophoretic separation of the small fragment, the only colonies obtained have the structure of the expression plasmid. The plasmid is tetracycline resistant due to reformation of a complete tetracycline resistance gene at the *Bam*HI site. Derivation of each region of the plasmid is therefore as follows: the region clockwise from the single *Pst*I site, through the *Eco*RI and *Hind*III sites to the *Bam*HI site comes from pBR322; from the *Bam*HI site through the next *Eco*RI until the *Hind*III site is derived from pMB9, and finally the region marked RGH from the *Hind*III site to the *Pst*I site originated from pRGH-1.

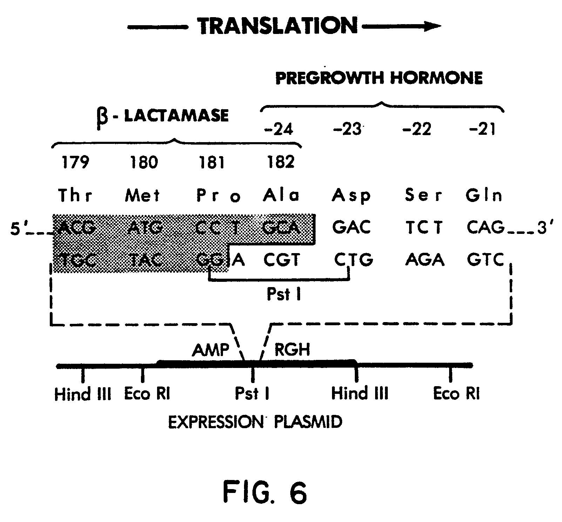

The complete DNA nucleotide sequences of the growth hormone insert and the beta-lactamase gene of pBR322 are known. See, Seeburg, P., et al., *supra*, and Sutcliffe, et al., *Proc. Nat. Acad. Sci. USA 75*, (1978). Reconstruction of these two sequences by joining them at their respective *Pst*I sites uniquely determines the phase of the pre-RGH sequence since the orientation of the RGH insert in pMB9 was determined. Figure 6 shows the sequence in the region of the *Pst*I site in pExRGH, and shows that the correct reading frame of preRGH is maintained. Therefore, the product expected to be coded for by this region of the plasmid is a chimeric poly-peptide consisting of the N-terminal 182 amino acids of beta-lactamase of which the first 23 are part of the pre-sequence, followed by 23 amino acids of the pre-sequence of RGH and 190 amino acids of RGH itself. Electrophoretic and immunochemical assay data as given in the following examples, demonstrate that such a protein is in fact synthesized in *E. coli* cells transformed by pExRGH.

As a practical matter, there are two general difficulties encountered with the expression of proteins coded by plasmids. First, as a general problem, the expressed protein must be obtained in pure form either from the growth medium or from cell extracts. A variety of techniques may be employed in combination. In certain instances, specific proteins are secreted into the medium. Such may be the case with beta-lactamase, a large portion of which is released into the medium when cells are converted to spheroplasts. However, the intracellular transport behavior of chimeric proteins cannot be predicted on

11

the basis of the behavior of either of the proteins that make up its structure. Examination on sodium dodecylsulfate-polyacrylamide gels of the polypeptides synthesized in the minicell producing strain P678—54 containing pExRGH or pBR322 shows several differences as would be expected for two similar but different plasmids. Most strikingly, a new polypeptide of about 47,000 molecular weight is observed with pExRGH containing minicells. This is within experimental error of the expected molecular weight for the chimeric protein containing the beta-lactamase fragment and pre-RGH, 44,300. At the same time, minicells containing pExRGH fail to make two polypeptides corresponding to pre-beta-lactamase and beta-lactamase, which are present in minicells containing pBR322. The details of this experiment are found in Example 2.

The foregoing strategy is suitable for constructing an expression plasmid containing the human growth hormone gene. At present, only a fragment of the HGH gene has been cloned, comprising all of the structural gene of the hormone but the first 23 amino acids. The HGH fragment was inserted into a plasmid via HindIII linkers and may therefore be isolated with HindIII. See Seeburg et al,., *supra* and US application Serial No. 897,710. The ends may thus be modified by filling the unpaired region using a DNA polymerase-catalyzed reaction. The resulting blunt-ended HGH fragment may be joined to a similarly blunt-ended beta-lactamase fragment created by HindII digestion. The technique for constructing an expression plasmid containing the entire HGH sequence will be similar in principle, once a full length sequence, coding for the hormone, is cloned.

Within the framework and general strategy of the described methods, it will be obvious to those skilled in the art that other expression transfer vectors for other proteins can be constructed. For example, the expression of human growth hormone will be achievable as generally described, as will be the expression of bovine, porcine and ovine growth hormones. Similarly, the described techniques may be employed in appropriate combination to achieve expression of the human insulin gene, as well as insulins of other species. Because the method is a general method, it may be applied to achieve the synthesis, by a microorganism, of any eucaryotic protein, barring obvious inoperability, such as a protein toxic to the microorganism.

In order to further illustrate the disclosed transfer vector, microorganisms and process, and to demonstrate their effectiveness in providing expression of the RGH gene and the rat insulin gene in a microorganism, the following examples are provided.

Example 1

The following methods have been used to construct expression transfer vectors and to test for chimeric protein synthesis.

A. Growth, isolation and purification of plasmids is accomplished by growing a suitable volume, e.g. 500 ml, of transformed cells in an overnight culture, with shaking, at 37°C. Optionally, cultures transformed by Co1El-derived plasmids such as pBR322, may be treated with 170 $\mu$g/ml chloramphenicol for 12—16 hours, to amplify the plasmid content. Cells are harvested by centrifugation, washed in 0.01 M Tris, 0.001 M EDTA, pH 8, resuspended in 10 ml cold 25% sucrose in 0.05 M Tris, 0.001 M EDTA, pH 8. Lysozyme, 2 ml, 5 mg/ml in 0.25 M Tris, pH 8, is added, followed by 4 ml 0.25 M EDTA pH 8. After 5—10 minutes incubation on ice, the cells are lysed by the addition of 0.1% (v/v) Triton X-100, Trademark, Rohmer and Haas Corp. Rutherford, N. J., in Tris-EDTA buffer, pH 8. After gentle mixing, the mixture is incubated about 15 minutes on ice. The lysate is centrifuged at 20,000 rpm for 25 min in the SS34 rotor of a Sorval centrifuge, Dupont instruments, Wilmington, Del.

The supernatant is decanted and further purified by chromatography on Sephadex G-100, Trademark Pharmacia Chemical Corp., Uppsala, Sweden, eluted with 0.2 M NaCl, 0.05 M Tris and 0.001 M EDTA, pH 8. The eluted DNA detected by absorbance at 260 nm, is precipitated by addition of 2 volumes cold 95% (w/v) ethanol, 0.03 volumes 3M sodium acetate and standing at —20°C overnight. The precipitate is collected, resuspended in Tris-EDTA buffer and centrifuged to equilibrium in CsCl containing ethidium bromide or propidium iodide. A 1/2 in×2 in centrifuge tube contains 2.2 g CsCl, 2.1 ml sample and 0.15 ml propidium iodide, 2 mg/ml in water. The tubes are centrifuged at 36,000 rpm for 18—24 hr at 20°C in a SW39 rotor, Beckman Instrument Co., Fullerton, Calif., or the equivalent. Fractions containing DNA are passed through a cation exchange column to remove propidium iodide and dialyzed against 0.01 M Tris, 0.001 M EDTA, pH 8 and 0.3 M sodium acetate to remove residual CsCl.

B. Transformation of *E. coli* RR-1 is carried out by growing a 10 ml culture in L-broth overnight at 37°C, diluting 1:50 by volume and growing 25 ml diluted culture until about 2—5×10[8] cells/ml are present (mid-log phase). The cells are harvested by centrifugation resuspended in 25 ml 10 mM NaCl, centrifuged again and resuspended in 25 ml 30 mM cold $CaCl_2$. The cells are kept at ice temperature 20 minutes, then harvested cold and resuspended in 1 ml 30 mM $CaCl_2$. To 0.2 ml cells are added 0.1 ml DNA, 10 pg/ml in 30 mM $CaCl_2$, 0.01 M Tris, 0.001 M EDTA, pH 7.5. The mixture is incubated on ice for 1 hour, then transferred to 42°C for 90 sec, then transferred back to ice. A rich medium such as L-broth, 3 ml, is added and the cells grown at 37°C for 3—12 hours before plating.

Transformation of *E. coli* X1776 is carried out similarly, as described in US application Serial No. 897,709.

C. Restriction endonucleases are available from a variety of commercial sources, as described in Ullrich, A. et al., *supra* and by Seeburg, P. H. et al., *supra*, and in US applications 897,709 and 897,710. These references also described incubation conditions for restriction endonuclease cleavage of DNA.

D. T4 DNA ligase may be obtained commercially from Bethesda Research Labs, Rockville, Md. The conditions for DNA joining are described in Ullrich, A. et al., supra, Seeburg, P. H. et al., *supra*, Sgaramella et al., *supra* and US applications Serial No. 897709 and 897,710.

E. Gel electrophoresis was performed as described by Dingman, C. W. and Peacock, A. C., *Biochemistry 7*, 659 (1968) and by Maniatis, T. et al., *Biochemistry 14*, 3787 (1975).

F. Antibody and antigen purification, for immunoassay, was sometimes necessary to rule out potential interference caused by any impurities present in the material used as antigen, either directly or to immunize the source animals. Affinity chromatography was employed, in which the column stationary phase was Sepharose 4B, Trademark Pharmacia Chemical Corp., Uppsala, Sweden, having antibody or antigen covalently bound by cyanogen bromide treatment. See March, S.C., et al., *Anal. Biochem. 60*, 149 (1974). The sample to be purified was loaded on the column in phosphate-buffered saline, washed with 15 column volumes of the same buffer, 5 column volumes of 0.05 M sodium acetate, pH 4.5, and eluted with 0.05 M glycine-HCl, pH 2.2.

G. A radioimmunassay was used to detect a chimeric protein comprising insulin or growth hormone. Lysates of cells transformed by an expression plasmid were tested, either as crude lysates or after partial purification by standard techniques. A dilution series of the lysate was made, to which antibody was added against bovine insulin (Miles Laboratories, Elkhardt, Ind.), against insulin C-peptide (See Yanaihara, C., et al., *Symposium on Proinsulin, Insulin and C-Peptide*, Tokushima, Japan, 1978, coordinated by Otsuka Pharmaceutical Co., Ltd.) or against rat growth hormone (prepared by immunizing male Rhesus monkeys against purified rat growth hormone). The labeled antigens added were [125I]-labeled insulin (New England Nuclear Corp., Boston, Mass.), [125I]-labeled tyrosylated rat C-peptide (Yanaihara, et al., *supra*) or [125I]-labeled growth hormone. The assay was essentially a modification of the method of Morgan and Lazarow, *Diabetes 12*, 115 (1963), modified according to Kessler, S. W., *J. Immunol. 115*, 1617 (1975) in which *Staphyloccocus Aureus* C cells were used instead of a second antibody, to precipitate antigen-antibody complexes.

H. Immune screening for expression was performed essentially as described by Erlich, H. A., et al., *Cell 10*, 681 (1978) or by Broome, S. and Gilbert W., *Proc. Nat. Acad. Sci. USA 75*, 2746 (1978).

Example 2

Analysis of labeled proteins synthesized by transformed cells. Proteins were labeled by addition [35S]-methionine, or of [3H] or [14C]-labeled amino acids to the growth medium. Crude or partially purified lysates were prepared and the proteins were fractionated by gel electrophoresis on sodium dodecyl sulfate-acrylamide gels as described by Laemmli, U.K., *Nature 227*, 680 (1970), or by two-dimensional gel electrophoresis-isoelectrofocusing described by O'Farrell, P. H., et al., *J. Biol, Chem, 250*, 4006 (1975). The first method fractionates proteins according to molecular weight, the second additionally fractionates on the basis of total ionic charge. Bands of radioactive proteins were detected by autoradiography as described by Ullrich, A., et al., *supra* or Seeburg, P. H. et al., *supra* and by US applications Serial No. 897,709 and 897,710.

To reduce the number of labeled proteins, lysates of transformed minicells were used in some experiments. The *E. coli* strains X1776 and P678—54 make minicells and are suitable for such studies.

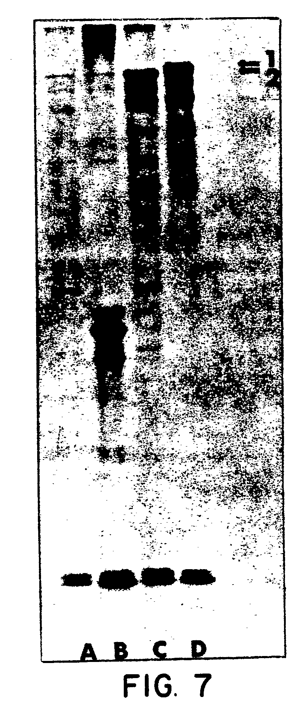

Fig. 7 shows gel electrophoresis bands resulting from X1776 minicells. Band (a) was obtained from non-transformed X1776. Band (b) was obtained from X1776 transformed with pBR322. Band (c) is X1776 transformed by pTS-1 and band (d) is X1176 transformed by pLRI-2. The band of beta-galactosidase indicated by arrow number 2, could be seen in pTS-1-transformed cells, while an additional band, arrow number 1, is seen in pLRI-2-transformed minicells. An 18% (w/v) acrylamide gel was used in the foregoing experiment. Resolution was improved in later experiments by the use of 6% (w/v) gels.

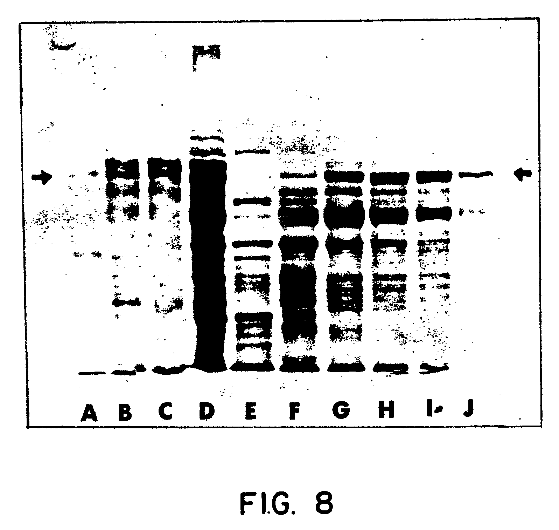

Fig. 8 shows the results of partial purification from whole cell lysates of *E. coli* DG-75 transformed cells. Cells were grown to an optimal density of <1.0 at 650 nm, 1 cm path length, harvested by centrifugation and frozen. Frozen cells were ground at 0°C in a precooled mortar with alumina, 7 g for cells from 1 liter of culture in the presence of phenylmethylsulfonyl fluoride (PMSF), a proteolytic enzyme inhibitor. Broken cells from 1 of culture were resuspended in 100 ml 0.02 M Tris pH 7.5, 0.01 MgCl$_2$, 0.1 M NaCl and 1 ml PMSF 1% (w/v) in dimethyl sulfoxide. Ammonium sulfate was added to 33% (w/v) final concentration. Precipitated protein was centrifuged and dialyzed against 40 mM Tris pH 8.0, 1 mM EDTA, 1 nM MgCl$_2$ and 5 nm beta-mercaptoethanol. DG-75 possesses a chromosomal beta-galactosidase gene which has a deletion near the N-terminal end that renders the enzyme inactive. However, transformation with pLRI-2 yields active enzymes, presumably due to complementation. In this instance, the beta-galactosidase which is a tetramer, can be active in the form of a mixed tetramer composed of individually inactive proteins. The tetramer is dissociated under the gel electrophoresis conditions. Bands (a) and (j) are markers of commercially purified beta-galactosidase (Boehringer-Mannheim Corp., New York, N.Y.) indicated by the arrows. Band (d) is the pattern of bands obtained from 33% (w/v) ammonium sulfate precipitable protein. The partially purified protein was fractionated by chromatography on DEAE-BioGel, Trademark BioRad Laboratories, Richmond, Calif. In 40 mM Tris, pH 8.0, 1 mM EDTA, 1 mM $MgCl_2$, 5 mM beta-mercaptoethanol, beta galactosidase was bound to the column but most of the chimeric galactosidase-pre-proinsulin was not. The proteins that did not bind under the above conditions were run on gels (b) and (c). The column was then eluted with a linear NaCl gradient. Beta-galactosidase activity was assayed and fractions eluting at and near the activity peak were analyzed in bands e—i. Peak activity was associated with band (g). As expected, the active peak was primarily composed of normal beta-galactosidase protein, but contained a minor compound of chimeric beta-galactosidase-proinsulin, as judged by the band density.

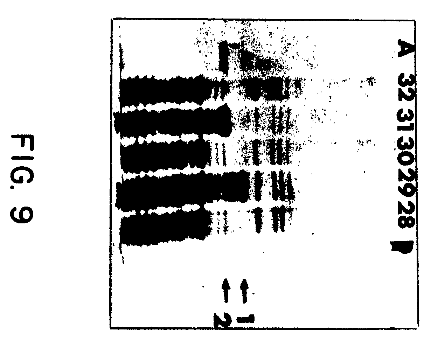

Fig. 9 shows gel electrophoresis in 18% (w/v) polyacrylamide gels, of unfractionated lysate proteins from single colony isolates of *E. coli* CSH20. The CSH20 strain was described by Miller, J., *supra*. There is a large deletion in the chromosomal beta-galactosidase gene in CSH20. Consequently no protein of that size is made in such cells unless they also carry a plasmid having the gene. A culture of CSH20 transformed with pLRI-2 was plated on XGal plates containing ampicillin. Both white and blue colonies were observed. Lane 28 shows the proteins banding pattern of a white colony isolate and lane 29 was protein from a blue colony. While lane 29 clearly displays the chimeric protein band at arrow 1, the isolate of lane 28 appears to have lost the ability to synthesize the chimeric protein. This behavior of expression plasmid-transformed cells is not explained. Similar behavior is observed in pTS-1 transformed cells. Lanes 30 and 31 represent single white and blue colony isolates, respectively. The presence of a substantial amount of synthesis of non-functional protein may be selected against under certain growth conditions, although the mechanism is not known. Lane 32 shows the banding pattern of a lysate of *E. coli* CSH17 (See Miller, J., *supra*) which has only a small deletion in beta-galactosidase. Lane A is a control of purified beta-galactosidase.

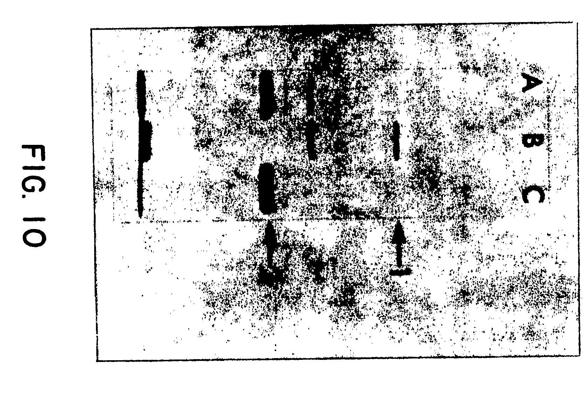

The data shown in Figs. 7—9 indicate the synthesis of a chimeric protein comprising a portion of the beta-galactosidase sequence and the proinsulin sequence. This conclusion was confirmed by nucleotide sequence analysis of the fused gene in pLRI-2. The nucleotide sequence was that coding for the expected chimeric protein. Fig. 10 shows gel electrophoresis of protein made by minicells of *E. coli* P678—54 transformed by pExRGH, or pBR322. The latter is shown in lane A. The dark band at arrow 2 is beta-lactamase, as shown by the control, Lane C, of extracellular secreted beta-lactamase. In lane B, the band of pattern of pExRGH is shown. The beta-lactamase band is absent and a new band is observed, at arrow 1. The estimated molecular weight of the new band was 47,000. The calculated molecular weight of the chimeric protein was 44,300, so that the measured value was well within the 10% error of the method.

Example 3

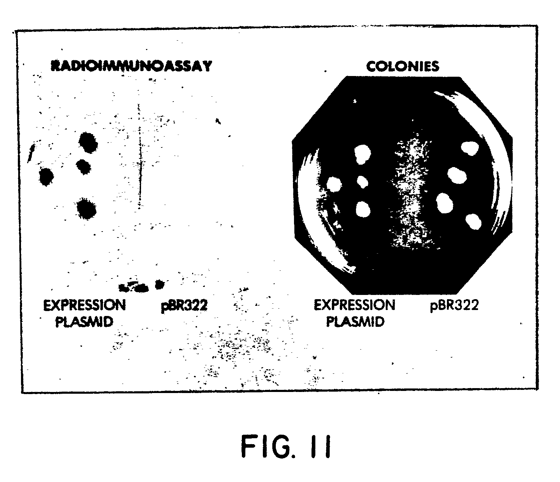

Fig. 11 shows the results of radioimmune screening of colonies transformed with pExRGH or pBR322. In this technique, referenced in Example 1, H., the colonies of transformed bacteria are partially lysed by exposure to chloroform vapor, then overlayed with a plastic film having anti-growth hormone antiserum, adsorbed thereto. The film is then exposed to [125I]-labeled anti-growth hormone, which may bind, as in an immunochemical "sandwich" assay, to sites occupied by any growth hormone, or an immunologically related chimeric protein. After washing, the film is then used to expose an autoradiographic plate. As shown in Fig. 11, all colonies transformed with pExRGH tested for positive growth hormone synthesis while those having pBR322 were negative.

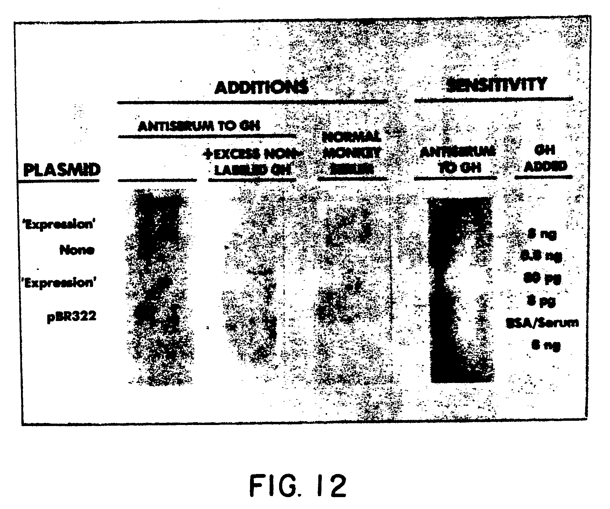

The reliability and sensitivity of the immunoscreening technique are demonstrated in Fig. 12. Under the heading "Additions", colonies of bacteria containing expression plasmid pExRGH, pBR322 or no plasmid were grown, as shown. In the left hand column, the screen, as previously described, was run, with the result previously shown. When excess unlabeled growth hormone was added, the [125I]-labeled antibody was completely bound, preventing any attachment to the colonies and demonstrating that the observed attachment was the result of anti-growth hormone and not some impurity in the antibody preparation. In a second control, normal (non-immune) monkey serum was used instead of anti-growth hormone. In the right hand column, indicated amounts of RGH were spotted on a test surface to determine sensitivity limits. Although a positive test was repeatedly observed with 8 ng, there was no reaction with lower levels. The reason for the unexpected cutoff is not known.

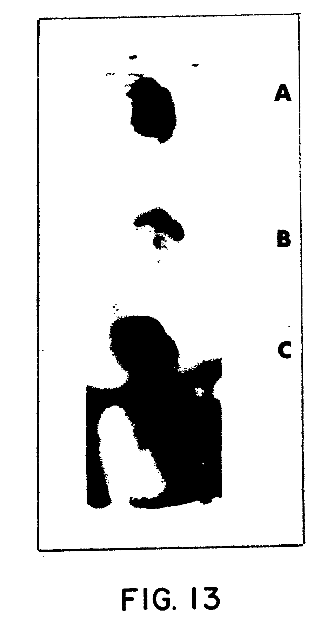

The immunoscreening test was used to confirm the identity of the beta-galactosidase-insulin chimeric protein of Figs. 7—9. Proteins isolated by gel electrophoresis were treated with CNBr, then spotted on immunoscreen gels. Results are shown in Fig. 13. At A, the large protein previously identified as a beta-galactosidase,-insulin was spotted. At B, beta-galactosidase itself, and at C, an insulin control. The result demonstrates that the chimeric protein contains the insulin sequence, and that the insulin sequence was reactive with anti-insulin antibody after CNBr treatment. Furthermore, in the case of the insulin immunoscreen assay, the sensitivity curve was normal, i.e., no cutoff was observed.

**Claims**

1. A DNA transfer vector comprising a nucleotide sequence which is at least partially cDNA and which codes for a eucaryotic protein, joined to a procaryotic control fragment, the reading frame of said nucleotide sequence coding for a eucaryotic protein being in register with the reading frame of said

## 0012494

control fragment, and the direction of transcription of said nucleotide sequence coding for a eucaryotic protein being the same as the direction of transcription of said control fragment.

2. The transfer vector of claim 1 wherein, upon transformation of a microorganism therewith, a chimeric protein is synthesized comprising an N-terminal amino acid sequence coded by the procaryotic control fragment joined to the eucaryotic protein as the C-terminal amino acid sequence.

3. The transfer vector of claim 1 or claim 2 wherein the eucaryotic protein is insulin.

4. The transfer vector of claim 3 wherein the eucaryotic protein is human insulin.

5. The transfer vector of claim 1 wherein the eucaryotic protein is growth hormone.

6. The transfer vector of claim 5 wherein the eucaryotic protein is human growth hormone.

7. The transfer vector of any of claims 1 to 4 wherein the procaryotic control fragment is that portion of the *E. coli* lactose operon comprising the initiator of transcription and initiator of translation regions thereof.

8. The transfer vector of any of claims 1, 2, 5 and 6 wherein the procaryotic control fragment comprises that portion of the *E. coli* beta-lactamase gene coding for the initiation of transcription and the initiation of translation regions thereof.

9. The transfer vector of any preceding claim wherein the procaryotic control fragment comprises a portion of a structural gene for a protein normally protected from intracellular degradation by compartmentalization.

10. The transfer vector of claim 9 wherein the procaryotic control fragment comprises a portion of the structural gene for a protein normally excreted into the growth medium.

11. The transfer vector of claim 9 wherein the procaryotic control fragment comprises a portion of the structural gene for beta-lactamase.

12. A transfer vector according to claim 2 wherein the transfer vector is the plasmid pLRI-2.

13. A transfer vector according to claim 2 wherein the transfer vector is the plasmid pExRGH.

14. A microorganism transformed by the transfer vector of any preceding claim.

15. A protein, synthesized by a microorganism according to claim 14, comprising the amino acid sequence of a eucaryotic protein.

16. The protein of claim 15 comprising the amino acid sequence of insulin.

17. The protein of claim 16 comprising the amino acid sequence of human insulin.

18. The protein of any of claims 15 to 17 synthesized by *Escherichia coli*, comprising additionally a portion of the N-terminal amino acid sequence of *Escherichia coli* beta-galactosidase.

19. A protein according to claim 15 comprising the amino acid sequence of a growth hormone.

20. The protein of claim 19 comprising the amino acid sequence of human growth hormone.

21. The protein of any of claims 15, 19 and 20 synthesized by *Escherichia coli*, comprising additionally a portion of the N-terminal amino acid sequence of *Escherichia coli* beta-lactamase.

22. A method for synthesizing a protein coded by a eucaryotic gene in a microorganism comprising the steps of,

a) selecting an expressable bacterial gene comprising a region controlling the initiation of transcription and initiation of translation,

b) providing a purified nucleotide sequence comprising the eucaryotic gene, wherein said nucleotide sequence comprises cDNA,

c) cleaving the bacterial gene and the nucleotide sequence thereby producing a procaryotic control fragment comprising a region controlling the initiation of transcription and initiation of translation and a nucleotide sequence fragment comprising the eucaryotic gene,

d) joining the control fragment and nucleotide sequence fragment in such orientation that the direction of transcription is the same for both fragments, transcription is initiated beginning with the control fragment and the reading frames of both fragments are in register, thereby producing a fused gene comprising a fragment of a bacterial gene and a fragment of a nucleotide sequence comprising a eucaryotic gene,

e) incorporating the fused gene into a transfer vector, thereby producing an expression transfer vector,

f) transforming a microorganism with the expression transfer vector,

g) selecting colonies of microorganisms containing the expression transfer vector, and

h) growing the selected microorganism containing the expression transfer vector under conditions permitting expression of the fused gene, whereby the eucaryotic gene is expressed.

23. The method of claim 22 wherein the bacterial gene contains a restriction site, the gene is cleaved in step c) with a restriction endonuclease, the control fragment thereby produced is modified by exonuclease digestion of unpaired ends and the modified control fragment is further modified by the addition of a synthetic restriction site linker sequence at one or both ends.

24. The method of claim 23 wherein the microorganism is the bacteria *Escherichia coli*, the bacterial gene is *E. coli* beta-galactosidase, the nucleotide sequence comprising a eucaryotic gene is the insulin fragment pAU-1 and the resulting expression fragment is pLRI-1.

25. The method of claim 22 wherein the nucleotide sequence comprising a eucaryotic gene has a restriction site, the nucleotide sequence is cleaved with a restriction endonuclease, the nucleotide sequence fragment comprising the eucaryotic gene produced in step c) is modified by exonuclease digestion to remove unpaired ends, and the modified fragment is further modified by the addition of synthetic restriction site nucleotide sequences at one or both ends.

26. The method of claim 25 wherein the microorganism is the bacterium *E. coli*, the eucaryotic gene fragment comprises the insulin gene fragment formed by combining pAU-1 and pAU-2 at their common Alul site and the expression transfer vector formed in step f) is the plasmid pLRI-2.

27. The method of claim 22 wherein the bacterial gene has a restriction site, the gene is cleaved in step c) with a restriction endonuclease, the nucleotide sequence comprising a eucaryotic gene has the same restriction site as in the bacterial gene, and the nucleotide sequence comprising a eucaryotic gene is cleaved in step c) with a restriction endonuclease to produce a nucleotide sequence fragment comprising the eucaryotic gene.

28. The method of claim 27 wherein the microorganism is the bacteria *Escherichia coli*, the bacterial gene comprises the beta-lactamase gene, the nucleotide sequence comprising a eucaryotic gene comprises the rat growth hormone gene and the expression transfer vector formed in step e) is the plasmid pExRGH.

29. The bacteria *Escherichia coli* strain CSH-20 containing and replicating plasmid pLRI-2.

30. The bacteria *Escherichia coli* strain P678—54 containing and replicating plasmid pExRGH.

**Revendications**

1. Vecteur de transfert d'ADN, caractérisé en ce qu'il comprend une séquence nucléotidique qui est au moins partiellement l'ADNc et qui code pour une protéine eucaryote, jointe à un fragment régulateur procaryote, le système de lecture de la séquence nucléotidique codant pour une protéine eucaryote étant en correspondance avec le système de lecture du fragment régulateur, la direction de la transcription de la séquence de nucléotides codant pour la protéine eucaryote étant la même que le direction de transcription du fragment régulateur.

2. Vecteur de transfert selon la revendication 1 caractérisé en ce que, après transformation d'un microorganisme à l'aide de ce vecteur, il est synthétisé une protéine chimérique comprenant une séquence N-terminale d'acides aminés codée par le fragment régulateur procaryote joint à la protéine eucaryote en tant que séquence C-terminale d'acides aminés.

3. Vecteur de transfert selon l'une quelconque des revendications 1 ou 2, caractérisé en ce que la protéine eucaryote est l'insuline.

4. Vecteur de transfert selon la revendication 3, caractérisé en ce que la protéine eucaryote est l'insuline humaine.

5. Vecteur de transfert selon la revendication 1, caractérisé en ce que la protéine eucaryote est l'hormone de croissance.

6. Vecteur de transfert selon la revendication 5, caractérisé en ce que la protéine eucaryote est l'hormone de croissance humaine.

7. Vecteur de transfert selon l'une quelconque des revendications 1 à 4, caractérisé en ce que le fragment régulateur procaryote est la partie de l'opéron lactose de *E. coli* comprenant ses sites de déclenchement de la transcription et de déclenchement de la traduction.

8. Vecteur de transfert selon l'une quelconque des revendications 1, 2, 5 et 6, caractérisé en ce que le fragment régulateur procaryote comprend la partie du gène bêta-lactamase de *E. coli* codant pour ses sites de déclenchement de la transcription et de déclenchement de la traduction.

9. Vecteur de transfert selon l'une quelconque des revendications 1 à 8, caractérisé en ce que le fragment régulateur procaryotique comprend une partie d'un gène de structure pour une protéine normalement protégée d'une dégradation intracellulaire par compartimentalisation.

10. Vecteur de transfert selon la revendication 9, caractérisé en ce que le fragment régulateur procaryote comprend une partie du gène de structure pour une protéine normalement excrétée dans le milieu de culture.

11. Vecteur de transfert selon la revendication 9, caractérisé en ce que le fragment régulateur procaryote comprend une partie du gène de structure pour la bêta-lactamase.

12. Vecteur de transfert selon la revendication 2, caractérisé en ce que le vecteur de transfert est le plasmide pLRI-2.

13. Vecteur de transfert selon la revendication 2, caractérisé en ce que le vecteur de transfert est le plasmide pExRGH.

14. Microorganisme transformé par le vecteur de transfert selon l'une quelconque des revendications 1 à 13.

15. Protéine synthétisée par un microorganisme selon la revendication 14 et comprenant la séquence d'acides aminés d'une protéine eucaryote.

16. Protéine selon la revendication 15, comprenant la séquence d'acides aminés de l'insuline.

17. Protéine selon la revendication 16, comprenant la séquence d'acides aminés de l'insuline humaine.

18. Protéine selon l'une quelconque des revendications 15 à 17, synthétisée par *Escherichia coli*,

**0 012 494**

caractérisée en ce qu'elle comprend en outre une partie de la séquence N-terminale d'acides aminés de la bêta-galactosidase d'Escherichia coli.

19. Protéine selon la revendication 15, comprenant la séquence d'acides aminés d'une hormone de croissance.

20. Protéine selon la revendication 19, comprenant la séquence d'acides aminés de l'hormone de croissance humaine.

21. Protéine selon l'une quelconque des revendications 15, 19 ou 20, synthétisée par Escherichia coli, caractérisée en ce qu'elle comprend en outre une partie de la séquence N-terminale d'acides aminés de la bêta-lactamase d'Escherichia coli.

22. Procédé de synthèse d'une protéine codée par un gène eucaryote dans un microorganisme, caractérisé en ce qu'il comprend les étapes consistant:

a) à sélectionner un gène bactérien exprimable comprenant un site régulateur du déclenchement de la transcription et du déclenchement de la traduction,

b) à créer une séquence nucléotidique purifiée comprenant le gène eucaryote, cette séquence de nucléotides comprenant de l'ADNc,

c) à couper le gène bactérien et la séquence de nucléotides en formant un fragment régulateur procaryote comprenant un site de régulation du déclenchement de la transcription et du déclenchement de la traduction et un fragment de séquence de nucléotides comprenant le gène eucaryote,

d) à assembler le fragment régulateur et le fragment de la séquence nucléotidique selon une orientation telle que la direction de la transcription soit la même pour les deux fragments, que la transcription soit déclenchée en commençant par le fragment régulateur et que les systèmes de lecture des deux fragments soient en coïncidence, en produisant ainsi un gène fusionné comprenant un fragment d'un gène bactérien et un fragment d'une séquence nucléotidique comprenant un gène eucaryote,

e) à incorporer le gène fusionné dans un vecteur de transfert, produisant ainsi un vecteur de transfert actif,

f) à transformer un microorganisme à l'aide du vecteur de transfert actif,

g) à choisir des colonies de microorganismes contenant le vecteur de transfert actif et

h) à faire croître le microorganisme choisi contenant le vecteur de transfert actif dans des conditions permettant l'expression du gène fusionné, ce qui exprime le gène eucaryote.

23. Procédé selon la revendication 22, caractérisé en ce que le gène bactérien contient un site de restriction, que le gène est coupé dans l'étape c) à l'aide d'une endonucléase de restriction, que le fragment régulateur ainsi produit est modifié aux extrémités non-appariées, par une digestion par une exonucléase, et que le fragment régulateur modifié subit une modification supplémentaire par l'addition d'une séquence synthétique, liant un site de restriction à l'une ou aux deux extrémités.

24. Procédé selon la revendication 23, caractérisé en ce que le microorganisme est la bactérie Escherichia coli, le gène bactérien est la bêta-galactosidase de E. coli, la séquence de nucléotides comprenant un gène eucaryote est le fragment insuline pAU-1 et le fragment actif résultant est pLRI-1.

25. Procédé selon la revendication 22, caractérisé en ce que la séquence de nucléotides comprenant un gène eucaryote possède un site de restriction, que la séquence de nucléotides est coupée par une endonucléase de restriction, que le fragment de séquence de nucléotides comprenant le gène eucaryote produit dans l'étape c) est modifié par une digestion par une exonucléase pour éliminer les extrémités non-appariées, et que le fragment modifié subit une modification supplémentaire par l'addition, à l'une ou aux deux extrémités, de séquences synthétiques de nucléotides de site de restriction.

26. Procédé selon la revendication 25, caractérisé en ce que le microorganisme est la bactérie E. coli, que le fragment de gène eucaryote comprend le fragment de gène de l'insuline formé en combinant pAU-1 et pAU-2 sur leur site commun AluI, et que le vecteur de transfert actif formé dans l'étape f) est le plasmide pLRI-2.

27. Procédé selon la revendication 22, caractérisé en ce que le gène bactérien possède un site de restriction, que le gène est clivé dans l'étape c) avec une endonucléase de restriction, que la séquence de nucléotides comprenant un gène eucaryote possède le même site de restriction que dans le gène bactérien, et que la séquence de nucléotides comprenant un gène eucaryote est coupée dans l'étape c) par une endonucléase de restriction pour produire un fragment de séquence de nucléotides comprenant le gène eucaryote.

28. Procédé selon la revendication 27, caractérisé en ce que le microorganisme est la bactérie Escherichia coli, que le gène bactérien comprend le gène de la bêta-lactamase, que la séquence de nucléotides comprenant un gène eucaryote comprend le gène de l'hormone de croissance du rat et que le vecteur de transfert actif formé dans l'étape e) est le plasmide pExRGH.

29. Bactérie Escherichia coli, souche CSH-20, contenant et repliquant le plasmide pLRI-2.

30. Bactérie Escherichia coli, souche P678—54, contenant et repliquant le plasmide pExRGH.

17

**0012494**

## Patentansprüche

1. DNA-Transfervektor mit einer Nukleotidsequenz, die zumindest teilweise cDNA ist und für ein eukaryontisches Protein, gekoppelt an ein prokaryontisches Kontrollfragement, codiert, wobei das Ablesemuster (reading frame) der für ein eukaryontisches Protein codierenden Nukleotidsequenz im Einklang steht mit dem Ablesemuster des Kontrollfragments, und die Richtung der Transkription der für ein eukaryontisches Protein codierenden Nukleotidsequenz die gleiche ist wie die Richtung der Transkription des Kontrollfragments.

2. Transfervektor nach Anspruch 1, dadurch gekennzeichnet, daß bei einer damit erfolgten Transformation eines Mikroorganismus ein chimäres Protein synthetisiert wird, das eine N-terminale Aminosäuresequenz enthält, die durch das prokaryontische Kontrollfragment, das an das eukaryontische Protein als C-terminale Aminosäuresequenz gekoppelt ist, codiert ist.

3. Transfervektor nach Anspruch 1 oder 2, dadurch gekennzeichnet, daß das eukaryontische Protein Insulin ist.

4. Transfervektor nach Anspruch 3, dadurch gekennzeichnet, daß das eukaryontische Protein menschliches Insulin ist.

5. Transfervektor nach Anspruch 1, dadurch gekennzeichnet, daß das eukaryontische Protein Wachstumshormon ist.

6. Transfervektor nach Anspruch 5, dadurch gekennzeichnet, daß das eukaryontische Protein menschliches Wachstumshormon ist.

7. Transfervektor nach einem der Ansprüche 1 bis 4, dadurch gekennzeichnet, daß das prokaryontische Kontrollfragment der Teil des *E. coli* Laktose-Operons ist, der den Initiator der Transkription und Initiator der Translationsbereiche davon enthält.

8. Transfervektor nach einem der Ansprüche 1, 2, 5 und 6, dadurch gekennzeichnet, daß das prokaryontische Kontrollfragment den Teil des *E. coli* Beta-Laktamase-Gens enthält, der für die Initiierung der Transkription und die Initiierung der Translationsbereiche davon codiert ist.

9. Transfervektor nach einem der vorhergehenden Ansprüche, dadurch gekennzeichnet, daß das prokaryontische Kontrollfragment einen Teil eines Strukturgens für ein Protein enthält, das normalerweise gegen intrazellularen Abbau durch Aufteilung geschützt ist.

10. Transfervektor nach Anspruch 9, dadurch gekennzeichnet, daß das prokaryontische Kontrollfragment einen Teil des Strukturgens für ein Protein enthält, das normalerweise in das Wachstumsmedium ausgeschieden wird.

11. Transfervektor nach Anspruch 9, dadurch gekennzeichnet, daß das prokaryontische Kontrollfragment einen Teil des Strukturgens für Beta-Laktamase enthält.

12. Transfervektor nach Anspruch 2, dadurch gekennzeichnet, daß der Transfervektor das Plasmid pLRI-2 ist.

13. Transfervektor nach Anspruch 2, dadurch gekennzeichnet, daß der Transfervektor das Plasmid pExRGH ist.

14. Mikroorganismus, transformiert durch den Transfervektor nach einem der vorhergehenden Ansprüche.

15. Protein, synthetisiert durch einen Mikroorganismus nach Anspruch 14, enthaltend die Aminosäuresequenz eines eukaryontischen Proteins.

16. Protein nach Anspruch 15, dadurch gekennzeichnet, daß es die Aminosäuresequenz von Insulin enthält.

17. Protein nach Anspruch 16, dadurch gekennzeichnet, daß es die Aminosäuresequenz von menschlichem Insulin enthält.

18. Protein nach einem der Ansprüche 15 bis 17, synthetisiert durch *Escherichia coli*, zusätzlich enthaltend einen Teil der N-terminalen Aminosäuresequenz von *Escherichia coli* Beta-Galaktosidase.

19. Protein nach Anspruch 15, enthaltend die Aminosäuresequenz eines Wachstumshormons.

20. Protein nach Anspruch 19, enthaltend die Aminosäuresequenz von menschlichem Wachstumshormon.

21. Protein nach einem der Ansprüche 15, 19 und 20, synthetisiert durch *Escherichia coli*, enthaltend zusätzlich einen Teil der N-terminalen Aminosäuresequenz von *Escherichia coli* Beta-Laktamase.

22. Verfahren zur Synthese eines durch ein eukaryontisches Gen codierten Proteins in einem Mikroorganismus, enthaltend die Verfahrensstufen

    (a) Auswählen eines ausdrückbaren Bakteriengens, das einen Bereich zur Kontrolle der Initiierung der Transkriptions und der Initiierung der Translation enthält,

    (b) Bereitstellen einer gereinigten Nukleotidsequenz, die ein eukaryontisches Gen enthält und wobei die Nukleotidsequenz cDNA enthält,

    (c) Spaltung des Bakteriengens und der Nukleotidsequenz unter Bildung eines prokaryontischen Kontrollfragments, das einen Bereich zur Kontrolle der Initiierung der Transkription und Initiierung der Translation enthält, und eines Nukleotidsequenzfragmentes, das das eukaryontische Gen enthält,

18

(d) Kuppeln des Kontrollfragmentes und des Nukleotidsequenz-Fragments in einer solchen Orientierung, daß die Richtung der Transkription für beide Fragmente die gleiche ist, die Transkription beginnend mit dem Kontrollfragment initiiert wird, und die Ablesemuster der beiden Fragmente im Einklang stehen, wobei ein verschmolzenes Gen gebildet wird, das ein Fragment eines bakteriellen Gens und ein Fragment einer Nukleotidsequenz, die ein eukaryontisches Gen enthält, enthält,

(e) Einbauen des verschmolzenen Gens in einen Transfervektor, wodurch ein Ausdrucks-Transfervektor (expression transfer vector) gebildet wird,

(f) Transformierung eines Mikroorganismus mit dem Ausdrucks-Transfervektor,

(g) Auswählen von Mikroorganismenkolonien, die den Ausdrucks-Transfervektor enthalten, und

(h) Züchten der ausgewählten Mikroorganismen, die den Ausdrucks-Transfervektor enthalten, unter Bedingungen, die den Ausdruck des verschmolzenen Gens ermöglichen, wodurch das eukaryontische Gen ausgedrückt wird.

23. Verfahren nach Anspruch 22, dadurch gekennzeichnet, daß das bakterielle Gen eine Restriktionsstelle enthält, das Gen in Verfahrensschritt (c) mit einer Restriktions-Endonuklease gespalten wird, das dabei gebildete Kontrollfragment durch Exonuklease-Aufschluß ungepaarter Enden modifiziert wird, und das modifizierte Kontrollfragment durch Zugabe einer synthetischen Restriktionsstellen-Verbindungssequenz an einem oder beiden Enden weiter modifiziert wird.

24. Verfahren nach Anspruch 23, dadurch gekennzeichnet, daß der Mikroorganismus das Bakterium *Escherischia coli* ist, das bakterielle Gen *E. coli* Beta-Galaktosidase ist, die ein eukaryontisches Gen enthaltende Nukleotidsequenz das Insulinfragment pAU-1 ist, und das resultierende Ausdrucksfragment pLRI-1 ist.

25. Verfahren nach Anspruch 22, dadurch gekennzeichnet, daß die Nukleotidsequenz ein eukaryontisches Gen als Restriktionsstelle enthält, die Nukleotidsequenz mit einer Restriktions-Endonuklease gespalten wird, das in Verfahren (c) gebildete eukaryontische Gen enthaltende Nukleotidsequenz-Fragment durch Exonuklease-Aufschluß zur Entfernung ungepaarter Enden modifiziert wird, und das modifizierte Fragment durch Zugabe synthetischer Restriktionsstellen-Nukleotidsequenzen an einem oder beiden Enden weiter modifiziert wird.

26. Verfahren nach Anspruch 25, dadurch gekennzeichnet, daß der Mikroorganismus das Bakterium *E. coli* ist, das eukaryontische Genfragment das durch Vereinigung von pAU-1 und pAU-2 an ihrer gemeinsamen AluI-Stelle gebildete Insulinfragment enthält und der in Verfahrensschritt (f) gebildete Ausdrucks-Transfervektor das Plasmid pLRI-2 ist.

27. Verfahren nach Anspruch 22, dadurch gekennzeichnet, daß das bakterielle Gen eine Restriktionsstelle hat, das Gen in Verfahrensschritt (c) mit einer Restriktions-Endonuklease gespalten wird, die ein eukaryontisches Gen enthaltende Nukleotidsequenz die gleiche Restriktionsstelle wie im bakteriellen Gen besitzt und die ein eukaryontisches Gen enthaltende Nukleotidsequenz in Verfahrensschritt (c) mit einer Restriktions-Endonuklease gespalten wird unter Bildung eines Nukleotidsequenz-Fragments, das das eukaryontische Gen enthält.

28. Verfahren nach Anspruch 27, dadurch gekennzeichnet, daß der Mikroorganismus das Bakterium *Escherichia coli* ist, das bakterielle Gen das Beta-Laktamasegen enthält, die ein eukaryontisches Gen enthaltende Nukleotidsequenz das Rattenwachstumshormon-Gen enthält, und der in Verfahrensschritt (e) gebildete Ausdrucks-Transfervektor das Plasmid pExRGH ist.

29. Der *Escherichia coli*-Baktereinstamm CSH-20, der Plasmid pLRI-2 enthält und repliziert.

30. Der *Escherichia coli*-Bakterienstamm P678—54, der Plasmid pExRGH enthält und repliziert.

FIG. 1

FIG. 2

Eco RI + Hind III digestion

$2 \times 10^4$ d

$2.7 \times 10^6$ d

$3.8 \times 10^6$ d

$7.7 \times 10^6$ d

T4 DNA Ligase

Transformation

Selection on Ampicillin/XGal plates

Screening for correct size ($6.5 \times 10^6$ d)

pTS-1

FIG. 3

3

FIG. 4

# CONSTRUCTION OF PLASMID
# TO EXPRESS GROWTH HORMONE

FIG. 5

— TRANSLATION →

**PREGROWTH HORMONE**

**β - LACTAMASE**

|  |  |  | -24 | -23 | -22 | -21 |
|---|---|---|---|---|---|---|
| 179 | 180 | 181 | 182 |  |  |  |
| Thr | Met | Pro | Ala | Asp | Ser | Gln |

5'---ACG   ATG   CCT   GCA   GAC   TCT   CAG___3'

TGC   TAC   GGA   CGT   CTG   AGA   GTC

Pst I

AMP      RGH

Hind III   Eco RI          Pst I          Hind III        Eco RI

EXPRESSION PLASMID

# FIG. 6

FIG. 7

FIG. 8

FIG. 9

FIG. 10

FIG. 11

FIG. 12

FIG. 13